# EUROPEAN PATENT SPECIFICATION

(11) **EP 1 570 819 B1**
(45) Date of publication and mention of the grant of the patent: **20.04.2011**
(21) Application number: 04077823.5
(22) Date of filing: 14.10.2004
(51) Int. Cl.: A61F 5/44, A61F 5/453

(54) **Urine drainage device**
Harnableitvorrichtung
Dispositif pour l'évacuation d'urine

(30) Priority: 01.03.2004 PL 36572604
(43) Date of publication of application: 07.09.2005
(73) Proprietor: LARKIS Spólka z o.o., 32-410 Dobczyce (PL)
(72) Inventor: Zurek, Aleksy, 32-415 Czaslaw (PL); Dobrowolski, Zygmunt, 31-106 Krakow (PL)
(74) Representative: Bartula-Toch, Marta

(56) References cited:
- WO-A-02/094126
- US-A- 5 013 308
- US-A- 6 113 582
- US-B1- 6 635 037

## Description

The object of the present invention is an urine drainage device to be used by men who do not have voluntary control over their urination due to various diseases, injuries and surgical interventions and thus urinating continuously.

In prior art uretheral catheters introduced into the urethra are known. Such devices exclude the skin contact with urine, thus eliminating irritation, however the problem of internal infections remains that can be transmitted into the bladder and kidneys. Such infections are difficult to control and dangerous for elder men who often suffer from other diseases too. In addition, introducing a catheter is difficult, time-consuming and uncomfortable.

Another common solution is an external catheter disposed about the penis and its terminus is coupled with a flexible tubing to the urine reservoir in the form of a bag fastened to the patient's leg. The reservoir has a drain for periodic drainage. The catheter takes the form of a condom that slides out from the penis when the patient is urinating or moving, thus making that solution unusable.

To clear such defect the catheter is fixed to the penis or its surrounding areas with a glue or adhesive tapes, thus causing skin irritation, chafes and wounds and providing no firm coupling.

An important disadvantage of this solution is penis loading with the urine drainage tubing that cause phallodynia and then testalgia.

When changing from sitting to standing position during urination, an air bubble forms that momentarily interrupts urine discharge resulting in penis wetting, catheter loosening and sliding. However, when a patient makes uncontrolled position changes during sleep, the catheter is pulled out by the urine discharge tubing.

Such inconvenience is cleared in part in the Polish utility pattern No. 42016. A set for a man suffering from urinary incontinence comprises a felt lined belt joined with buttons to lugs of cylindrical disk having a groove on its perimeter. A condom is disposed onto the disk and secured in the groove with a clip. The disk has four lugs spaced asymmetrically over its perimeter to enable fastening to the belt and legs with thigh straps. An inflexible adapter with socket cap inclined at angle from 0° to 45° to the sleeve is placed in the condom terminus. One end of the tubing is inserted onto the adapter, while another end is coupled to the urine reservoir that takes the shape of polygon of nine sides at least. The reservoir is vertically divided into two chambers with a pressure weld over the length greater than the half of total length of the reservoir. There is a drain closed with plug in the bottom of the reservoir.

Disadvantages of such solution are hard inflexible elements that stiffen the set and compress the patient's body. Another inconvenience results from the catheter itself being a flabby condom.

Another solution is a men's urine collecting belt described in the Polish patent description No. Ru 48 648. This comprises a hip belt with a pair of flexible suspenders which in turn are coupled to a catheter. The catheter has a truncated cone rigid part, bent along its axis and externally coated with a flexible coating. The rigid part has a threaded cap with hole. The thread allows the urine drainage tubing to be attached. The other end of the tubing is coupled to the urine reservoir that is fastened with suspenders to the patient's shin. The bottom part of the reservoir along with a drain pipe and valve is bent upward at 180°.

A disadvantage of that device is rigid catheter that is uncomfortable and compresses the patient's body

A common disadvantage of all known devices is an urine discharge opening centrally located in an external catheter, thus preventing all urine discharge into the reservoir. This cause permanent wetting of the penis that leads to lesions and discomfort to the patient. In addition, the problem of air bubble hampering urine discharge also remains unsolved.

The use of condom-like catheters leads also to other troubles, for example prepuce suction into the drainage channel, this blocking urine discharge into the reservoir and leading to prepuce necrosis after prolonged urine wetting of the penis and prepuce.

Patent description No. US 6635037 B1 presents a male urinary incontinence management device, consisting of a bearing portion and an external condom catheter, connected through the upper part with a base support portion, adjustably attached to the patient's body with bearing elements. The base support portion has a shape close to a truncated cone with external aperture. The greater base of the support portion is placed on the pubic part of male body , while the smaller base is connected through an aperture with the catheter resembling the shape of the penis. The catheter consists of a body shaped according to the shape of a penis, a pear-shaped extended part according to the shape of the glans, and a pipe-shaped ending where the urinary duct is connected.

The bearing portion consists of a belt and a system of adjustable straps with conventional combining and adjusting elements that may comprise bands, hooks, velcros, buttons and clasps.

Patent description WO 02/094126 A discloses a solution concerning a drainage bag for human body fluids. It has a reusable outer bag containing an inner single-use pouch, containing a relevant amount of a hydrophilic material, preferably with antibacterial or antiseptic properties. The inner pouch has an inlet tube inserted from above, through which the body fluid flows. The inlet tube above the pouch has a three-way valve that opens or closes the flow of body fluid, and makes collection of body fluid samples possible.

The present invention comprises urine drainage devices used for the same purposes and of similar design, fastening and application.
The terms: fist invention, second invention and third invention are used for editorial purposes only, to facilitate reading and understanding of this description, and follows the order of presentation.

The object of the present invention is an external catheter disposed onto the penis. The catheter is a flexible and soft tube connector bent in its upper part at the angle α from 80° to 140° or blinded from the top, with side aperture enclosed with a flexible cap, temporarily or permanently coupled to a fastening element or fastening elements.

The catheter is of self-supporting design, i.e. it maintains its shape by not fitting exactly to the penis shape as in the case of condom-like catheters of the same or similar wall thickness. The inside dimensions of the catheter are slightly greater than the penis size.

The bottom part of the catheter is blinded and has a tightly connected flexible urine tubing coupled to the urine reservoir that has an additional vent tube.

The side aperture is preferably coupled with a ring to the cap having two or more lugs.

Preferably, the cap and/or its lugs have such fastening elements as a lace eye, clasp, Velcro, clamp or snap. preferably, a strap fastening element has on both or one end such fastening elements as a lace eye, clasp, Velcro, clamp or snap.

Preferably, the fastening element is in the form of a flap of material with a hole slid over the catheter.

The object of the second invention is an external catheter disposed onto the penis. The catheter is a flexible and soft tube connector bent in its upper part at the angle α from 80° to 140° or blinded from the top, with side aperture enclosed with a flexible cap, temporarily or permanently coupled to a fastening element or fastening elements.

The catheter is of self-supporting design, i.e. it maintains its shape by not fitting exactly to the penis shape as in the case of condom-like catheters of the same or similar wall thickness. The inside dimensions of the catheter are slightly greater than the penis size.

The bottom part of the catheter is blinded and has from two to four tightly connected flexible urine tubes tightly coupled to the urine reservoir.

The side aperture is preferably coupled with a ring to the cap having two or more lugs.

preferably, the cap and/or its lugs have such fastening elements as a lace eye, clasp, Velcro, clamp or snap.

Preferably, a strap fastening element has on both or one end such fastening elements as a lace eye, clasp, Velcro, clamp or snap.

Preferably, the fastening element is in the form of a flap of material with a hole slid over the catheter.

The object of the third invention is an external catheter disposed onto the penis. The catheter is a flexible and soft tube connector bent in its upper part at the angle α from 80° to 140° or blinded from the top, with side aperture enclosed with a flexible cap, temporarily or permanently coupled to a fastening element or fastening elements. The catheter is of self-supporting design, i.e. it maintains its shape by not fitting exactly to the penis shape as in the case of condom-like catheters of the same or similar wall thickness. The inside dimensions of the catheter are slightly greater than the penis size. The bottom part of the catheter is blinded and has two or three tightly connected flexible urine tubes that merge into a single tubing tightly coupled to the urine reservoir.

The side aperture is preferably coupled with a ring to the cap having two or more lugs.

Preferably, the cap and/or its lugs have such fastening elements as a lace eye, clasp, Velcro, clamp or snap. Preferably, a strap fastening element has on both or one end such fastening elements as a lace eye, clasp, Velcro, clamp or snap.

Preferably, the fastening element is in the form of a flap of material with a hole slid over the catheter.

The main advantage of the solutions presented above is a loose flexible catheter of self-supporting design that provides the penis with sufficient and continuous contact with the air, and urine discharge system enabling urine to be discharged without backflow resulting from the presence of air bubble as in previous solutions. In the first solution this is achieved by a dedicated vent tube, while in the second and third solutions by employing at least one of urine tubes not discharging urine at the moment. By use of several urine tubes coupled to the catheter tip and appropriately spaced it is possible to provide proper urine discharge in any laying position, thus improving patient's relaxation and undisturbed sleep.

Another advantage is also a different way of catheter fastening to the body compared to those previously used, avoiding mechanical loading of the penis as Well as skin irritation and chafes.

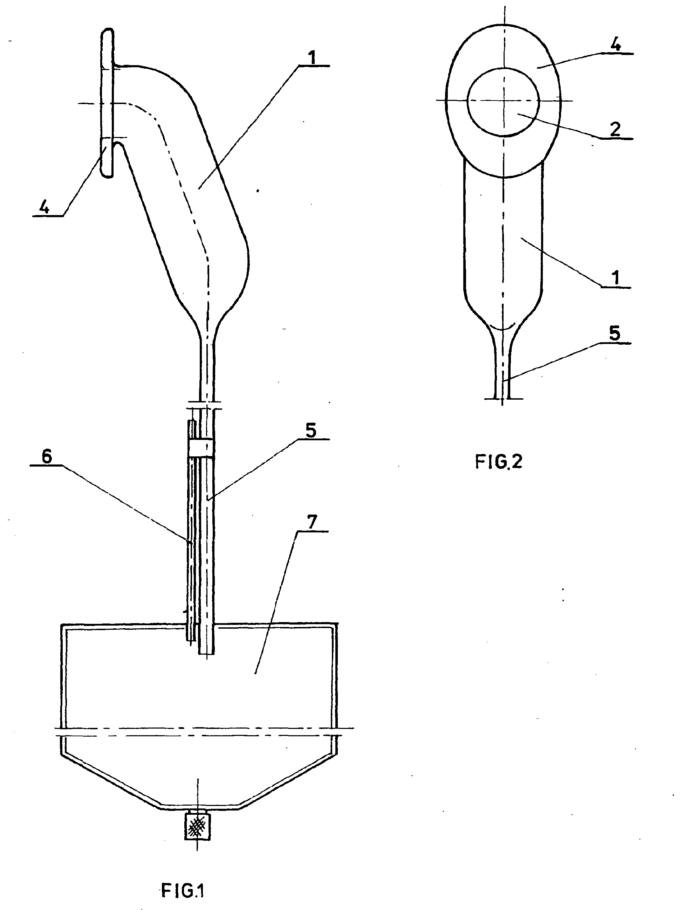

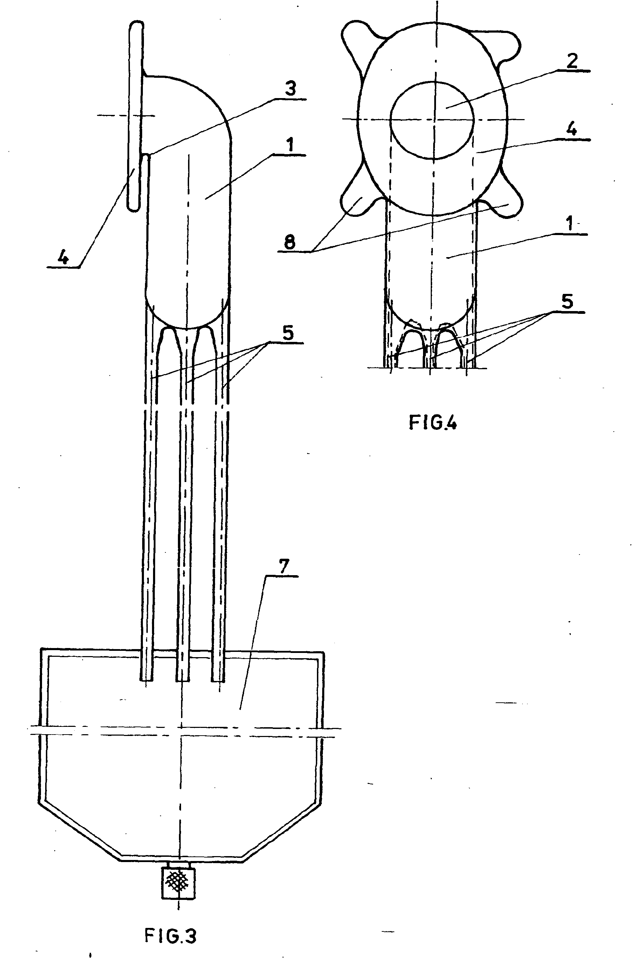

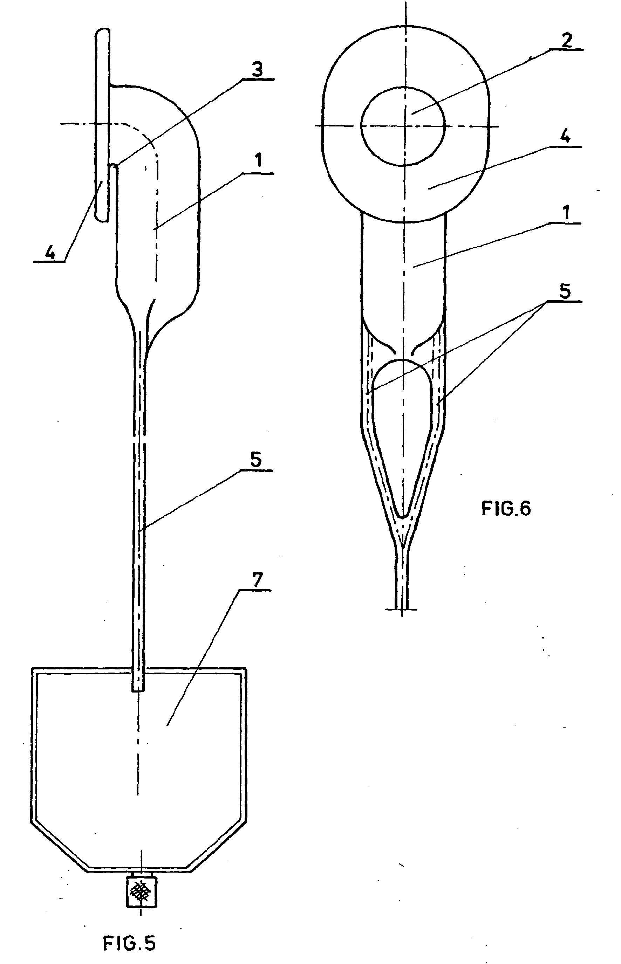

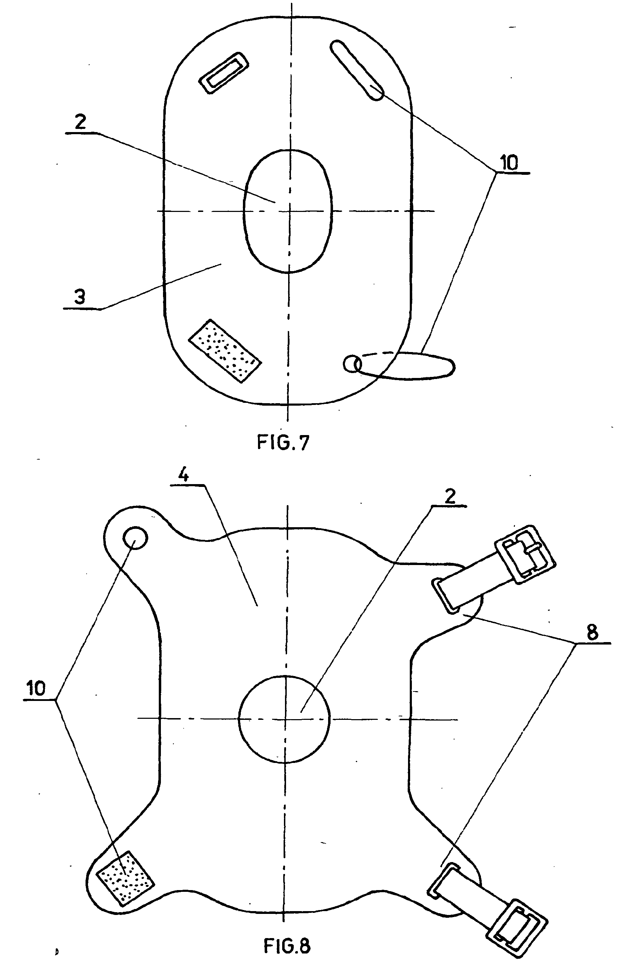

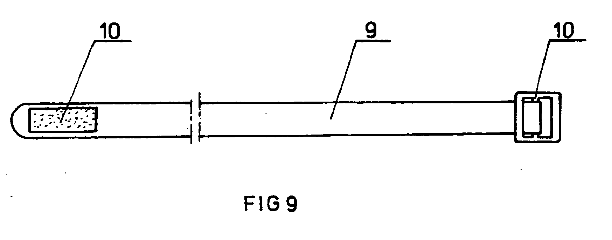

The solutions of the present inventions are presented in the following figures: Fig. 1 shows side view of the device of the first invention, Fig. 2 - view of the catheter 1, from the side aperture 2, with upper section of the urine discharge tubing 5 - according to the first invention, Fig. 3 - shows side view of the device of the second invention, Fig. 4 - view of the catheter 1, from the side aperture 2 with upper sections of urine tubes 5 - according to the second invention, Fig. 5 - shows side view of the device of the third invention, Fig. 6 - view of the catheter 1 from side 2 with upper sections of urine discharge tubes 5, merged into a single urine tubing 5 according to the third invention, Fig. 7 - principal view of the cap 3 of the catheter 1 the same for the all three inventions - as described in Example I, Fig. 8 - principal view of the cap 3 of the catheter 1 - the same for the all three inventions - as described in Example II, Fig. 9 - view of a strap fastening element 9 with fastenings 10 on both ends, for the solution of all three inventions - as described in Example I

### ExampleI

According to the first invention the device comprises a catheter 1 in the form of a flexible tube connector blinded in its upper part and of size slightly greater then those of the non erected penis. The catheter 1 is of self-supporting design, i.e. it maintains the same shape before and after deposition onto the penis, and is bent along the angle α = 110°. Its walls are made from a flexible and soft material and are of 0,5 to 2 mm in thickness. The catheter 1 has an oval-like side aperture 2 in close vicinity of the blinded tip. The aperture minor axis is slightly greater than the diameter of non erected penis. Aperture 2 is enclosed with an oval-like flexible soft cap 4 of surface area considerable greater than that of aperture 2. The catheter 1 narrows into a sleeve and is tightly and permanently coupled to a flexible urine discharge tubing 5 of small diameter, that in turn is tightly joined to the urine reservoir 7 that has a drain hole with a valve. There is a flexible vent tube 6 of diameter considerably smaller than that of the urine discharge tubing 5 and running from the urine reservoir 7 alongside the urine discharge tubing 5. The vent tube 6 is longitudinally joined to the urine discharge tubing 5 and ends with an open outlet over approximately over 1/3 of its length.

The cap 4 has in its four apparent corners the fastenings 10 in the form of a small slit, small hole with a lace eye and a small-size Velcro element. The fastening elements 9 in the form of narrow straps are coupled to the cap 4 its own fasteners 10. There are fasteners 10 on the ends of the fastening elements 10 in the form of a clamp and Velcro element to be coupled to similar Velcro element of the cap 4. The end of one of strap fastening elements 9 is threaded through the slit of the cap 4 and joined to the fastening element 9 with the clamp fastener 10.

The device of the first invention is designed primarily for ambulant patients. After disposing the catheter 1 onto the penis, it is coupled to the body with fastening elements 9, while the urine tubing 5 and the vent tube 6 are placed along one of the legs and the urine reservoir 7 is tied to the calf. The vent tube 6 allows air to be removed from the urine reservoir 7 and avoids urine bubbling and backflow in the urine discharge tubing 5

### Example II

According to the second invention the device emprises a catheter 1 in the form of a flexible soft tube connector of size slightly greater then those of the non erected penis. The catheter 1 is of self-supporting design, i.e. it maintains the same shape before and after deposition onto the penis. Its walls are made from a moderately flexible and soft material and are of 0,5 to 2 mm in thickness. The catheter 1 is of elbow-like shape bent at angle α = 90° with forming a short ring 3 around side aperture 2 of circular-like shape and size slightly greater than the diameter of non erected penis. The ring 3 ends with a flexible soft cap 4 of rectangular-like shape. Its shorter horizonal sides are or arc shape. At passing from straight to arc shape the cup 4 has lugs 8 of various shapes, as shown in Figure, with fastenings 10 in the form of short strap with a buckle, short strap with a clasp, Velcro element. They are coupled to the cap 4 with its corresponding fasteners 10, while the fastening elements 9 in the form of flexible straps are joined to the patient's body.

The catheter 1 is blinded in its lower part and tightly coupled to three flexible urine discharge tubes 5 of small diameter, that are tightly joined to the urine reservoir 7. The outlets of the urine tubes 5 are located in the bottom of the catheter 1 at corners of an apparent triangle. The urine tubes 5 are joined over its entire length, thus avoiding kink of the other leg when the patient is laying.

The device of the second invention is designed primarily for bedridden patients. After disposing the catheter 1 onto the penis, it is coupled to the body with fastening elements 9, while the urine tubes 5 and the urine reservoir 6 are tied to the patient's leg or lead outside the bed. By spacing the urine tubes 5 at triangle corners of catheter 1, one or two of them - depending on the patient's position - can discharge urine, while the remaining one or two perform the role of vent tube.

### Example III

According to the third invention the device comprises a catheter 1 in the form of a flexible soft tube connector of size slightly greater then those of the non erected penis. The catheter 1 is of self-supporting design, i.e. it maintains the same shape before and after deposition onto the penis and is bent along the angle α = 90°. Its walls are made from a moderately flexible and soft material and are of 0,8 to 2,2 mm in thickness. The upper part of the catheter 1 is blinded and below it there is a circular side aperture 2 of size slightly greater than the diameter of non erected penis. Apertures is enclosed with a short ring 3 finished with a flexible soft cap 4 of considerable greater size and of rectangular shape with rounded corners.

The catheter 1 is blinded in its lower part and tightly coupled to two flexible urine discharge tubes 5 of small diameter, that merge into a single urine tubing 5 below approximately half of its length. The tubing in turn is tightly joined to the urine reservoir 7.

The catheter 1 is coupled to the patient's body with a flap fastening element 9 not shown here.

The third invention comprises an all-purpose solution that can be utilized both by bedridden and ambulant patients.

## Claims

1. An urine discharge device consisting of external catheter disposed onto the penis and temporarily coupled to the body and joined with a small-diameter flexible tubing to the urine reservoir, wherein the catheter (1) is flexible and soft and of self-supporting design, i.e. it maintains its shape before and after disposing onto the penis, and its upper part is an elbow bent at the angle α from 80 to 140° or is blinded, and have side aperture (2) finished with a flexible and soft cap (4), temporarily or permanently coupled to fastening elements (9), and in addition the catheter (1) has in its lower part a tightly coupled flexible urine discharge tubing (5), joined to the urine reservoir (7), that has an additional vent tube (6).

2. The device of claim 1, wherein side aperture (2) is coupled to the cap (4) with a short ring (3), while the cap (4) preferably has two lugs (8) and the cap (4) and/or its lugs (8) have fastenings (10) to connect fastening elements (9).

3. The device of claim 2, wherein the fastening (10) is a lace eye, clasp, Velcro, clamp or snap, while a trap fastening element (9) has preferably on its one of both ends a fastener (10) being a lace eye, clasp, Velcro, clamp, catch or snap.

4. The device of claim 1, wherein the catheter (1) flap aperture is slid over the fastening element (9).

5. An urine discharge device consisting of external catheter disposed onto the penis and temporarily coupled to the body and joined with a small-diameter flexible tubing to the urine reservoir, wherein the catheter (1) is flexible and soft and of self-supporting design, i.e. it maintains its shape before and after disposing onto the penis, and its upper part is an elbow bent at the angle α from 80 to 140° or is blinded, and have side aperture (2) finished with a flexible and soft cap (4), temporarily or permanently coupled to fastening elements (9), and in addition the catheter (1) has in its lower part from two to four tightly coupled flexible urine discharge tubes (5), tightly joined to the urine reservoir (7).

6. the device of claim 5, wherein side aperture (2) is coupled to the cap (4) with a short ring (3), while the cap (4) preferably has two lugs (8) and the cap (4) and/or its lugs (8) have fastenings(10) to connect fastening elements (9).

7. The device of claim 6, wherein the fastening (10) is a lace eye, clasp, Velcro, clamp or snap, while a trap fastening element (9) has preferably on its one of both ends a fastener (10) being a lace eye, clasp, Velcro, clamp, catch or snap.

8. The device of claim 5, wherein the catheter (1) flap aperture is slid over the fastening element (9).

9. An urine discharge device consisting of external catheter disposed onto the penis and temporarily coupled to the body and joined with a small-diameter flexible tubing to the urine reservoir, wherein the catheter (1) is flexible and soft and of self-supporting design, i.e. it maintains its shape before after disposing onto the penis, and its upper part is an elbow bent at the angle α from 80 to 140° or is blinded, and have side aperture (2) finished with a flexible and soft cap (4), temporarily or permanently coupled to fastening elements (9), and in addition the catheter (1) has in its lower part from two to three tightly coupled flexible urine discharge tubes (5), that below merge into a single urine tubing (5) tightly joined to the urine reservoir (7).

10. the device of claim 9, wherein side aperture (2) is coupled to the cap (4) with a short ring (3), while the cap (4) preferably has two lugs (8) and the cap (4) and/or its lugs (8) have fastenings(10) to connect fastening elements (9).

11. The device of claim 10, wherein the fastening (10) is a lace eye, clasp, Velcro, clamp or snap, while a trap fastening element (9) has preferably on its one of both ends a fastener (10) being a lace eye, clasp, Velcro, clamp, catch or snap.

12. The device of claim 9, wherein the catheter (1) flap aperture is slid over the fastening element (9).

## Patentansprüche

1. Vorrichtung zur Harnableitung mit einem an den Penis anzubringenden und mit an dem Körper trennbar zu befestigenden Befestigungselementen externen Katheter, verbunden mit einer dünnen und elastischen Rohrleitung mit dem Harnbehälter, **gekennzeichnet dadurch, dass** das Katheter (1) die Form eines elastischen und gleichzeitig weichen Stutzen mit einer selbsttragenden Bauart hat, die die vorgegebene Gestalt sowohl vor als auch nach der Anbringung an den Penis einhält; der Stutzen ist in seinem oberen Teil in einem Winkel von 80 bis 140° knieartig gebogen bzw. oben geschlossen, mit einer Seitenöffnung (2), gemündet mit einem elastischen und gleichzeitig weichen Flansch (4), der mit Befestigungselement bzw. -elementen (9) trennbar oder untrennbar verbunden ist; des Weiteren besitzt das Katheter (1) in seinem unteren Teil eine abgeleitete und dicht verbundene, elastische Harnrohrleitung (5), die mit einem Harnbehälter (7) verbunden ist, von dem eine zusätzliche Entlüftungsrohrleitung (6) abgeleitet ist.

2. Die Vorrichtung gem. Anspruch 1 **gekennzeichnet dadurch, dass** die Seitenöffnung (2) mit dem Flansch (4) mit einem kurzen Ring (3) verbunden ist, wobei der Flansch (4) günstigerweise zwei Feder (8) besitzt und der Flansch (4) und/oder seine Feder (8) besitzen Verbindungselemente (10) zur Verbindung mit Befestigungselementen (9).

3. Die Vorrichtung gem. Anspruch 2 **gekennzeichnet durch** Verbindungselement (10) in Form einer Schleife, Klammer, Klettverschluss, eines Klemmeelements, Zapfelements bzw. Rastelements, wobei das befestigende Bandelement (9) an einem bzw. zwei Enden ein Verbindungselement (10) in Form einer Schleife, Klammer, Klettverschluss, eines Klemmeelements, Zapfelements bzw. Rastelements besitzt

4. Die Vorrichtung gem. Anspruch 1 **gekennzeichnet dadurch, dass** an das Katheter (1) ein befestigendes Flügelelement (9) mit seiner Öffnung aufgebracht ist.

5. Vorrichtung zur Harnableitung mit einem an den Penis anzubringenden und mit an dem Körper trennbar zu befestigenden Befestigungselementen externen Katheter, verbunden mit einer dünnen und elastischen Rohrleitung mit dem Harnbehälter, **gekennzeichnet dadurch, dass** das Katheter (1) die Form eines elastischen und gleichzeitig weichen Stutzen mit einer selbsttragenden Bauart hat, die die vorgegebene Gestalt sowohl vor als auch nach der Anbringung an den Penis einhält; der Stutzen ist in seinem oberen Teil in einem Winkel α von 80 bis 140° knieartig gebogen bzw. oben geschlossen, mit einer Seitenöffnung (2), gemündet mit einem elastischen und gleichzeitig weichen Flansch (4), der mit Befestigungselement bzw. -elementen (9) trennbar oder untrennbar verbunden ist; des Weiteren besitzt das Katheter (1) in seinem unteren Teil abgeleitete und dicht verbundene Harnrohrleitungen (5) in einer Zahl von zwei bis vier, die mit dem Harnbehälter (7) dicht verbunden sind.

6. Die Vorrichtung gem. Anspruch 5 **gekennzeichnet dadurch, dass** die Seitenöffnung (2) mit dem Flansch (4) mit einem kurzen Ring (3) verbunden ist, wobei der Flansch (4) günstigerweise zwei Feder (8) besitzt und der Flansch (4) und/oder seine Feder (8) besitzen Verbindungselemente (10) zur Verbindung mit Befestigungselementen (9).

7. Die Vorrichtung gem. Anspruch 6 **gekennzeichnet durch** Verbindungselement (10) in Form einer Schleife, Klammer, Klettverschluss, eines Klemmeelements, Zapfelements bzw. Rastelements, wobei das befestigende Bandelement (9) an einem bzw. zwei Enden ein Verbindungselement (10) in Form einer Schleife, Klammer, Klettverschluss, eines Klemmeelements, Zapfelements bzw. Rastelements besitzt

8. Die Vorrichtung gem. Anspruch 5 **gekennzeichnet dadurch, dass** an das Katheter (1) ein befestigendes Flügelelement (9) mit seiner Öffnung aufgebracht ist.

9. Vorrichtung zur Harnableitung mit einem an den Penis anzubringenden und mit an dem Körper trennbar zu befestigenden Befestigungselementen externen Katheter, verbunden mit einer dünnen und elastischen Rohrleitung mit dem Harnbehälter, **gekennzeichnet dadurch, dass** das Katheter (1) die Form eines elastischen und gleichzeitig - weichen Stutzen mit einer selbsttragenden Bauart hat, die die vorgegebene Gestalt sowohl vor als auch nach der Anbringung an den Penis einhält; der Stutzen ist in seinem oberen Teil in einem Winkel α von 80 bis 140° knieartig gebogen bzw. oben geschlossen, mit einer Seitenöffnung (2), gemündet mit einem elastischen und gleichzeitig weichen Flansch (4), der mit Befestigungselement bzw. -elementen (9) trennbar oder untrennbar verbunden ist; des Weiteren besitzt das Katheter (1) in seinem unteren Teil abgeleitete und dicht verbundene zwei bzw. drei elastische Harnrohrleitungen (5), die unten zu einer einzigen elastischen Harnrohrleitung (5) zusammengelegt sind, mit dem Harnbehälter (7) dicht verbunden.

10. Die Vorrichtung gem. Anspruch 9 **gekennzeichnet dadurch, dass** die Seitenöffnung (2) mit dem Flansch (4) mit einem kurzen Ring (3) verbunden ist, wobei der Flansch (4) günstigerweise zwei Feder (8) besitzt und der Flansch (4) und/oder seine Feder (8) besitzen Verbindungselemente (10) zur Verbindung mit Befestigungselementen (9).

11. Die Vorrichtung gem. Anspruch 10 **gekennzeichnet durch** Verbindungselement (10) in Form einer Schleife, Klammer, Klettverschluss, eines Klemmeelements, Zapfelements bzw. Rastelements, wobei das befestigende Bandelement (9) an einem bzw. zwei Enden ein Verbindungselement (10) in Form einer Schleife, Klammer, Klettverschluss, eines Klemmeelements, Zapfelements bzw. Rastelements besitzt

12. Die Vorrichtung gem. Anspruch 9 **gekennzeichnet dadurch, dass** an das Katheter (1) ein befestigendes Flügelelement (9) mit seiner Öffnung aufgebracht ist.

## Revendications

1. Dispositif destiné à évacuer l'urine, comportant un cathéter extérieur installé sur le pénis, fixé au corps de façon temporaire avec des éléments de fixation, associé à un réservoir d'urine avec un tube fin et flexible, ledit dispositif étant **caractérisé en ce que** le cathéter (1) est sous la forme d'une tubulure autoporteuse flexible et molle à la fois, gardant sa forme aussi bien avant qu'après son installation sur le pénis, coudée en sa partie supérieure à l'angle a de 80° à 140° ou obturée à son extrémité supérieure, et comportant une ouverture latérale (2) se fermant par une collerette flexible et molle à la fois (4), associée de façon temporaire ou définitive à un élément de fixation ou à des éléments de fixation (9), et le cathéter (1) en sa partie inférieure possède un tube flexible d'évacuation d'urine introduit à l'intérieur du cathéter de manière étanche (5), et associé à un réservoir d'urine (7), duquel sort un tube de mise à l'air libre supplémentaire (6).

2. Dispositif selon la revendication 1 **caractérisé en ce que** l'ouverture latérale (2) est associée avec la collerette (4) avec un anneau court (3), et la collerette (4) possède comme avantage des languettes (8) en quantité de deux et la collerette (4) et/ou ses languettes (8) possède (possèdent) des éléments de jonction (10) pour assembler les éléments de fixation (9).

3. Dispositif selon la revendication 3 **caractérisé en ce que** l'élément d'attache (10) est une maille, une agrafe, un élément d'attache velcro, un élément de pince, un élément d'accrochage ou un élément de cliquet, et l'élément de fixation sous la forme d'une bande (9) possède comme avantage, à une ou à deux de ses extrémités, un élément d'attache (10) qui est une maille, une agrafe, un élément d'attache velcro, un élément de pince, un élément d'accrochage ou un élément de cliquet.

4. Dispositif selon la revendication 1 **caractérisé en ce que** sur le cathéter (1) est empiété, par son ouverture, un élément de fixation sous la forme d'une aile (9).

5. Dispositif destiné à évacuer l'urine, comportant un cathéter extérieur installé sur le pénis, fixé au corps de façon temporaire avec des éléments de fixation, associé à un réservoir d'urine avec un tube fin et flexible, ledit dispositif étant **caractérisé en ce que** le cathéter (1) est sous la forme d'une tubulure autoporteuse flexible et molle à la fois, gardant sa forme aussi bien avant qu'après son installation sur le pénis, coudée en sa partie supérieure à l'angle a de 80° à 140° ou obturée à son extrémité supérieure, et comportant une ouverture latérale (2) se fermant par une collerette flexible et molle à la fois (4), associée de façon temporaire ou définitive à un élément de fixation ou à des éléments de fixation (9), et le cathéter (1) en sa partie inférieure possède des tubes flexibles d'évacuation d'urine introduits à l'intérieur du cathéter de manière étanche (5), en quantité de deux à quatre, associés à un réservoir d'urine (7).

6. Dispositif selon la revendication 5 **caractérisé en ce que** l'ouverture latérale (2) est associée avec la collerette (4) avec un anneau court (3), et la collerette (4) possède comme avantage des languettes (8) en quantité de deux et la collerette (4) et/ou ses languettes (8) possède (possèdent) des éléments d'attache (10) pour assembler les éléments de fixation (9).

7. Dispositif selon la revendication 6 **caractérisé en ce que** l'élément d'attache (10) est une maille, une agrafe, un élément d'attache velcro, un élément de pince, un élément d'accrochage ou un élément de cliquet, et l'élément de fixation sous la forme d'une bande (9) possède comme avantage, à une ou à deux de ses extrémités, un élément d'attache (10) qui est une maille, une agrafe, un élément d'attache velcro, un élément de pince, un élément d'accrochage ou un élément de cliquet.

8. Dispositif selon la revendication 5 **caractérisé en ce que** sur le cathéter (1) est empiété, par son ouverture, un élément de fixation sous la forme d'une aile (9).

9. Dispositif destiné à évacuer l'urine, comportant un cathéter extérieur installé sur le pénis, fixé au corps de façon temporaire avec des éléments de fixation, associé à un réservoir d'urine avec un tube fin et flexible, ledit dispositif étant **caractérisé en ce que** le cathéter (1) est sous la forme d'une tubulure autoporteuse flexible et molle à la fois, gardant sa forme aussi bien avant qu'après son installation sur le pénis, coudée en sa partie supérieure à l'angle a de 80° à 140° ou obturée à son extrémité supérieure, et comportant une ouverture latérale (2) se fermant par une collerette flexible et molle à la fois (3), associée de façon temporaire ou définitive à un élément de fixation ou à des éléments de fixation (9), et le cathéter (1) en sa partie inférieure possède deux ou trois tubes flexibles d'évacuation d'urine introduits à l'intérieur du cathéter de manière étanche (5), qui au-dessous débouchent sur un tube unique d'évacuation d'urine flexible (5), associé de manière étanche à un réservoir d'urine (7).

10. Dispositif selon la revendication 9 **caractérisé en ce que** l'ouverture latérale (2) est associée avec la collerette (4) avec un anneau court (3), et la collerette (4) possède comme avantage des languettes (8) en quantité de deux et la collerette (4) et/ou ses languettes (8) possède (possèdent) des éléments d'attache (10) pour assembler les éléments de fixation (9).

11. Dispositif selon la revendication 10 **caractérisé en ce que** l'élément d'attache (10) est une maille, une agrafe, un élément d'attache velcro, un élément de pince, un élément d'accrochage ou un élément de cliquet, et l'élément de fixation sous la forme d'une bande (9) possède comme avantage, à une ou à deux de ses extrémités, un élément d'attache (10) qui est une maille, une agrafe, un élément d'attache velcro, un élément de pince, un élément d'accrochage ou un élément de cliquet.

12. Dispositif selon la revendication 9 **caractérisé en ce que** sur le cathéter (1) est empiété, par son ouverture, un élément de fixation sous la forme d'une aile (9).
